# EUROPÄISCHE PATENTSCHRIFT

(11) **EP 0 321 833 B1**
(45) Veröffentlichungstag und Bekanntmachung des Hinweises auf die Patenterteilung: **18.11.1993**
(21) Anmeldenummer: 88120794.8
(22) Anmeldetag: 13.12.1988
(51) Int. Cl.: C07D 249/12

(54) **Verfahren zur Herstellung von 4-Amino-1,2,4-triazol-5-onen**
Process for the preparation of 4-amino-1,2,4-triazol-5-ones
Procédé pour la préparation de 4-amino-1,2,4-triazol-5-ones

(30) Priorität: 22.12.1987 DE 3743493; 11.08.1988 DE 3827264
(43) Veröffentlichungstag der Anmeldung: 28.06.1989
(73) Patentinhaber: BAYER AG, 51368 Leverkusen (DE)
(72) Erfinder: Müller, Klaus-Helmut, Dr., D-4000 Düsseldorf 13 (DE); König, Klaus, Dr., D-5068 Odenthal (DE); Heitkämper, Peter, Dr., D-4047 Dormagen (DE)

(56) Entgegenhaltungen:
- EP-A- 433 215
- FR-A- 1 470 310
- EUROPEAN JOURNAL OF MEDICINAL CHEMISTRY - CHIMIA THERAPEUTICA, Band 18, Nr. 3, 1983, Seiten 215-220, Paris, FR; R. MILCENT et al.: "Synthèse et activité antibactérienne d'amino-4 triazol-1,2,4 ones-5 substituées"
- JOURNAL OF HETEROCYCLIC CHEMISTRY, Band 19, Juli/August 1982, Seiten 823-828, Provoh, Utah, US; K.H. PILGRAM: "5-Substituted-3-(substituted)phenyl-1,3,4-oxadiazolin-2(3H)-ones. Synthesis and reactions with nucleophilic reagents"
- The Chemistry of Heterocyclic Compounds;5 and 6 Membered Compounds with Nitrogen and Oxygen;page 274,275,281,282
- The Chemistry of Heterocyclic Compounds; Trianoles-1,2,4 page 482-483
- Z. Chem. 8, 221, 1968

## Beschreibung

Die Erfindung betrifft ein neues Verfahren zur Herstellung von 4-Amino-1,2,4-triazol-5-onen, welche Zwischenprodukte zur Herstellung von herbiziden Wirkstoffen sind.

Es ist bekannt, daß man 4-Amino-1,2,4-triazol-5-one erhält, wenn man Carbohydrazid mit Carbonsäuren thermisch cyclisiert (vgl. Chem. Ber. 98, 3025 [1965]). Der Nachteil dieses Verfahrens liegt darin, daß der Ringschluß erst nach längerer Reaktionszeit (10 Stunden) erfolgt und unter diesen Reaktionsbedingungen bereits Selbstkondensation des Carbohydrazids zum 4-Aminourazol erfolgt. Die Ausbeuten an gewünschtem Triazolon sind bei diesem Verfahren gering (16 % bis 22 %).

Weiterhin ist bekannt, daß man 4-Amino-1,2,4-triazol-5-one erhält, wenn man 1,5-Diacyl-carbohydrazide in Gegenwart von wäßrigem Alkali cyclisiert. Die hierfür erforderlichen 1,5-Diacylcarbohydrazide werden zuvor durch Erwärmen von Carbohydrazid mit Carbonsäuren hergestellt (vgl. Chem. Ber. 98, 3025 [1965]). Auch bei diesem Verfahren sind die Ausbeuten über beide Stufen nicht sehr hoch.

Weiterhin ist bekannt, daß man 4-Amino-1,2,4-triazol-5-one erhält, wenn man N^{β}-Acylcarbazinsäureester mit Hydrazinhydrat umsetzt (vgl. Chem. Ber. 98, 3025 [1965]). Nachteilig bei diesem Verfahren sind ebenfalls die geringen Ausbeuten, insbesondere wenn man berücksichtigt, daß die N^{β}-Acylcarbazinsäureester, welche als Ausgangsverbindungen benötigt werden, ebenfalls erst hergestellt werden müssen. Ein weiterer gravierender Nachteil dieser Methode besteht darin, daß diese Methode nur bestimmte (3-Methyl)-4-Amino-1,2,4-triazol-5-one zugänglich macht. Bereits mit der homologen Ethyl-Verbindung N^{β}-Propionylcarbazinsäureethylester findet unter gleichen Bedingungen keine Cyclisierung mehr statt.

Weiterhin ist bekannt, daß man 4-Amino-1,2,4-triazol-5-one der unten beschriebenen Formel (I) erhält, wenn man Carbohydrazid mit Orthoestern thermisch cyclisiert (vgl. Chem. Ber. 27, 55 [1894]; J. Prakt. Chem. [2] 52, 454 [1895]; Chem. Ber. 57, 1321 [1924]; Liebigs Ann. Chem. 475, 120 [1929] und Inorganic Synthesis 4, 32 [1953]).

Nachteilig bei diesem Verfahren ist, daß als Ausgangsverbindungen Orthoester erforderlich sind, welche selbst in mehrstufiger - über hydrolyseanfällige Zwischenstufen verlaufender - Synthese hergestellt werden müssen, was die Gesamtsynthese aus Kosten- und Umweltgründen wenig vorteilhaft macht.

Ein weiteres bekanntes Verfahren zur Herstellung von 4-Amino-1,2,4-triazol-5-onen besteht in der Umsetzung von Pinner-Salzen mit Carbazinsäureestern und der anschließenden Cyclisierung mit Hydrazinhydrat (vgl. Bull. Soc. Chim. France 1962, 1364; Eur. J. Med. Chem. - Chim. Ther. 1983, 215; Chimica Acta Turcica 7, 269 [1979]). Nachteilig bei diesem Verfahren ist die niedrige Gesamtausbeute und die Herstellung der Pinner-Salze, wozu Reaktionszeiten von mehreren Tagen benötigt werden und unter strengem Feuchtigkeitsausschluß gearbeitet werden muß (vgl. Ber. dtsch. chem. Ges. 16, 1643 [1883]; Organic Syntheses, Coll. Vol. I, 5 [1951]).

Es wurde nun gefunden, daß man 4-Amino-1,2,4-triazol-5-one der allgemeinen Formel (I), 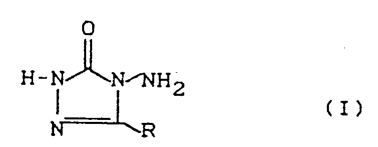
in welcher
- R: für geradkettiges oder verzweigtes Alkyl mit 1 bis 8 Kohlenstoffatomen steht
erhält, wenn man Acylhydrazide der Formel (II), 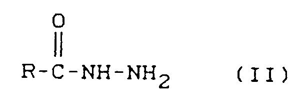
in welcher
- R: für geradkettiges oder verzweigtes Alkyl mit 1 bis 8 Kohlenstoffatomen steht
zunächst mit Phosgen gegebenenfalls in Gegenwart einer Base und gegebenenfalls in Gegenwart eines Verdünnungsmittels umsetzt und die so erhältlichen Oxadiazolinone der Formel (III), 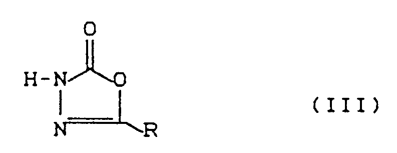
in welcher
- R: für geradkettiges oder verzweigtes Alkyl mit 1 bis 8 Kohlenstoffatomen steht
entweder ohne Isolierung direkt im "Eintopfverfahren" oder in einem separaten Reaktionsschritt mit Hydrazinhydrat gegebenenfalls in Gegenwart eines Verdünnungsmittels weiter umsetzt.

Es ist als ausgesprochen überraschend anzusehen, daß die erfindungsgemäße Umsetzung das gewünschte Produkt in hoher Ausbeute und Reinheit liefert, da aus dem Stand der Technik bekannt war, daß die Ringöffnung von Oxadiazolinonen in starkem Maße vom Substitutionsmuster abhängig ist und beispielsweise die entsprechenden 1-Alkyl-3-aryl-oxadiazolinone mit überschüssigem Hydrazin lediglich Aroylhydrazide liefern (vgl. Z. Chem. 8, 221 [1968]).

Als Vorteil des erfindungsgemäßen Verfahrens muß herausgestellt werden, daß die erforderlichen Ausgangsverbindungen der Formel (II) billige großtechnische Produkte sind, welche mit einfachsten Grundchemikalien weiter umgesetzt werden. Dabei ist insbesondere die Variante des erfindungsgemäßen Verfahrens, bei welcher die als Zwischenprodukte auftretenden Oxadiazolinone der Formel (III) direkt im "Eintopfverfahren" weiter umgesetzt werden, eine einfache und kostengünstige Möglichkeit, die gewünschten Endprodukte in hoher Ausbeute und Reinheit zu erhalten.

Die mit Hilfe des erfindungsgemäßen Verfahrens herstellbaren 4-Amino-1,2,4-triazol-5-one sind durch die Formel (I) allgemein definiert.

Vorzusweise herstellbar sind Verbindungen der Formel (I), bei welchen
- R: für geradkettiges oder verzweigtes Alkyl mit 1 bis 4 Kohlenstoffatomen steht.

Besonders bevorzugt herstellbar sind Verbindungen der Formel (I), bei welchen
- R: für Methyl oder Ethyl steht.

Verwendet man beispielsweise Acethydrazid, Phosgen und Hydrazinhydrat als Ausgangstoffe, so läßt sich der Reaktionsablauf des erfindungsgemäßen Verfahrens durch das folgende Formelschema darstellen: 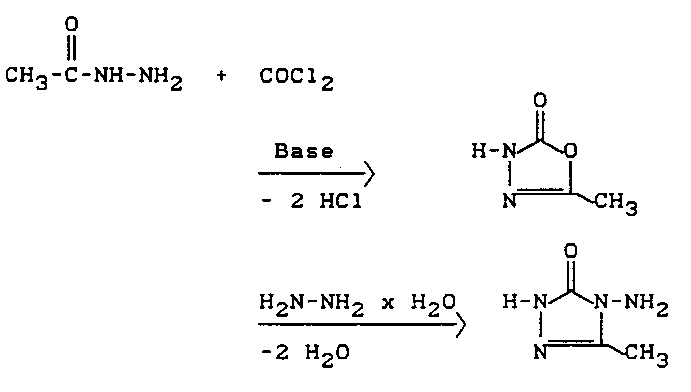
Die zur Durchführung des erfindungsgemäßen Verfahrens als Ausgangsstoffe benötigten Acylhydrazide sind durch die Formel (II) allgemein definiert. In dieser Formel (II) steht R vorzugsweise für diejenigen Reste, die bereits im Zusammenhang mit der Beschreibung der erfindungsgemäßen Stoffe der Formel (I) als bevorzugt für diesen Substituenten genannt wurden.

Die Acylhydrazide der Formel (II) sind allgemein bekannte Verbindungen der organischen Chemie.

Das erfindungsgemäße Verfahren wird vorzugsweise in Gegenwart eines geeigneten Verdünnungsmittels durchgeführt. Als solche kommen inerte organische Lösungsmittel oder wäßrige Systeme infrage. Hierzu gehören insbesondere aliphatische, alicyclische oder aromatisch, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Dichlorethan, Chloroform, Tetrachlorkohlenstoff oder Wasser.

Das erfindungsgemäße Verfahren kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphoniumbromid, Trimethyl-C₁₃/C₁₅-alkylammoniumchlorid, Dibenzyl-dimethyl-ammonium-methylsulfat, Dimethyl-C₁₂/C₁₄-alkyl-benzylammonium-chlorid, Tetrabutylammoniumhydroxid, 15-Krone-5, 18-Krone-6, Triethylbenzylammoniumchlorid, Trimethylbenzylammoniumchlorid oder Tris-[2-(2-methoxy-ethoxy)-ethyl]-amin.

Das erfindungsgemäße Verfahren kann außerdem in der 1. Stufe gegebenenfalls in Gegenwart einer geeigneten Base durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate oder -hydrogencarbonate, wie Natriumcarbonat, Kaliumcarbonat oder Natriumhydrogencarbonat, mit besonderem Vorzug verwendet man Natrium- oder Kaliumcarbonat als Base.

Die Reaktionstemperaturen können bei der Durchführung des erfindungsgemäßen Verfahrens in einem größeren Bereich variiert werden. Im allgemeinen arbeitet man bei Temperaturen zwischen -20 °C und +20 °C, vorzugsweise bei Temperaturen zwischen -10 °C und +10 °C in der ersten Stufe der Umsetzung sowie bei Temperaturen zwischen +20 °C und +120 °C, vorzugsweise bei Temperaturen zwischen +50 °C und +100 °C in der zweiten Stufe der Umsetzung.

Zur Durchführung des erfindungsgemäßen Verfahrens setzt man pro Mol an Acylhydrazid der Formel (II) im allgemeinen jeweils 1 bis 3 Mol, vorzugsweise 1 bis 2 Mol an Phosgen und gegebenenfalls Base sowie 1 bis 10 Mol, vorzugsweise 1 bis 5 Mol an Hydrazinhydrat ein.

Dabei geht man so vor, daß man zunächst das Acylhydrazid der Formel (II) gegebenenfalls zusammen mit der Hälfte der Base in dem Verdünnungsmittel vorlegt und dann solange Phosgen einleitet bis ein pH-Wert von < 3 erreicht ist, danach gibt man gegebenenfalls die zweite Hälfte Base zu und leitet wiederum Phosgen bis zum pH- Wert von 3 ein. Nach beendeter Reaktion entfernt man überschüssiges Phosgen durch Destillation oder Ausblasen mit Stickstoff, gibt weiteres Verdünnungsmittel und einen Überschuß Hydrazinhydrat zu und erwärmt für mehrere Stunden auf Rückflußtemperatur.

Die Aufarbeitung und Isolierung der Reaktionsprodukte erfolgt nach allgemein üblichen Methoden. Dabei kann es gegebenenfalls von Vorteil sein, die so erhältlichen 4-amino-1,2,4-triazol-5-one der Formel (I) von anhaftendem Kochsalz zu befreien, indem man sie auf üblichem Weg an der Aminogruppe derivatisiert, beispielsweise indem man sie mit Aldheyden oder Ketonen, wie beispielsweise Benzaldehyd oder Aceton in Azomethine überführt, wobei sich unlösliches Kochsalz leicht abfiltrieren läßt. Aus diesen Derivaten läßt sich die Azomethinschutzgruppe entweder mit üblichen Methoden wieder abspalten, sie lassen sich jedoch auch als solche als Ausgangsprodukte für weitere Umsetzungen direkt verwenden.

Die so erhältlichen 4-Amino-1,2,4-triazol-5-one sind wertvolle Zwischenprodukte, beispielsweise zur Synthese von herbiziden Wirkstoffen (vgl. z. B. US-PS 3 884 910 oder Deutsche Patentanmeldung P 37 19 575 vom 12.06.1987).

### Herstellungsbeispiele

### Beispiel 1

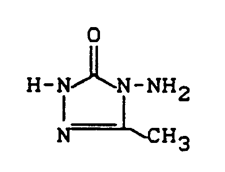

(zweistufiges Verfahren)
Zu 10 g (0.1 Mol) 5-Methyl-1,3,4-oxadiazol-2(3H)-on in 250 ml Wasser gibt man 18 g (0.36 Mol) Hydrazinhydrat, erhitzt ca. 12 Stunden auf Rückflußtemperatur, engt zur Trockne ein, nimmt den Rückstand in Ethanol auf, saugt unlösliches Produkt ab und trocknet es.

Man erhält 8,2 g (72 % der Theorie) an 4-Amino-3-methyl-1,2,4-triazol-5(4H)-on vom Schmelzpunkt 228 °C. 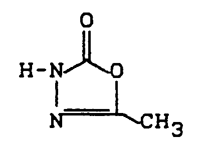
Zu einer Lösung aus 74,0 g (1.0 Mol) Acethydrazid und 53 g (0.5 Mol) wasserfreiem Natriumcarbonat in 300 ml Wasser leitet man unter Rühren und Kühlen bei -5 °C bis +5 °C einen kräftigen Phosgenstrom ein bis die Lösung schwach sauer reagiert (Methylorange als Indikator). Dann gibt man weitere 53 g (0.5 Mol) Natriumcarbonat zu und leitet ein zweites Mal Phosgen ein bis zum Indikatorumschlagpunkt. Zur Aufarbeitung entfernt man überschüssiges Phosgen durch Ausblasen mit Stickstoff, extrahiert dann 24 Stunden mit Essigester, engt die Essigester-Phase bis zur beginnenden Kristallisation ein, kühlt ab, filtriert, engt die Mutterlauge auf ein Viertel des Volumens ein, kühlt ab und gewinnt durch Filtration eine 2. Fraktion.

Nach Trocknen erhält man 80 g (80 % der Theorie) an 2-Methyl-1,3,4-oxadiazol-5(4H)-on vom Schmelzpunkt 112 °C. 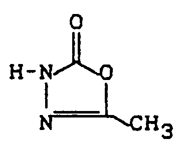
(alternative Herstellung)
Zu 450 g (4.5 Mol) Phosgen in 3000 ml wasserfreiem 1,2-Dichlorethan gibt man bei 10°C unter Kühlung und Rühren tropfenweise innerhalb von 45 bis 60 Minuten eine 50° bis 60°C warme Lösung von 222 g (3.0 Mol) wasserfreiem Acethydrazid in 600 ml wasserfreiem 1,2-Dichlorethan. Dabei sollte die Reaktionstemperatur der Mischung 25°C nicht übersteigen. Nach beendeter Zugabe wird auf 40°C bis 50°C erwärmt bis nach ca. 60 Minuten die Gasentwicklung nachläßt. Unter weiterem Einleiten von Phosgen (ca. 50 g pro Stunde) wird weiter auf Rückflußtemperatur erhitzt und 1 Stunde unter Rückfluß phosgeniert. Zur Aufarbeitung werden 1500 ml Lösungsmittel und überschüssiges Phosgen abdestilliert, heiß filtriert, daß Filtrat eingeengt und der Rückstand im Vakuum destilliert.

Man erhält 277 g (92 % der Theorie) an 2-Methyl-1,3,4-oxadiazol-5(4H)-on vom Siedepunkt 140° bis 143° C bei 16 mbar und von Schmelzpunkt 110°-111° C.

### Beispiel 2

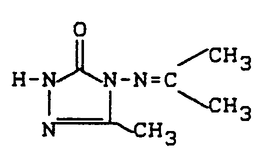

(Eintopfverfahren und Umsetzung zum Azomethin)
74 g (1.0 Mol) Acethydrazid, 53 g (0.5 Mol) Natriumcarbonat und eine Spatelspitze Methylorange werden in 300 ml Wasser gelöst und bei -5 °C bis +5 °C mit Phosgen versetzt, bis der Indikator umschlägt. Nach beendeter Kohlendioxid-Gasentwicklung gibt man weitere 53 g (0.5 Mol) Natriumcarbonat zu und leitet erneut Phosgen bis zum Indikatorumschlagpunkt ein. Nach beendeter Gasentwicklung vertreibt man überschüssiges Phosgen durch Ausblasen mit Stickstoff aus der Lösung, verdünnt mit 2,5 l Wasser, gibt 180 g (3.6 Mol) Hydrazinhydrat zu und erhitzt für 12 Stunden auf Rückflußtemperatur. Nach dem Abkühlen wird im Vakuum zur Trockne eingeengt, der Rückstand mit Ethanol verrührt, filtriert, der unlösliche Feststoff in 1,5 l Aceton aufgenommen, weitere 6 Stunden auf Rückflußtemperatur erhitzt und dann heiß filtriert. Das Filtrat wird eingeengt, durch Verreiben mit Ethanol kristallisiert, abgesaugt und getrocknet.

Man erhält 99 g (64 % der Theorie) an 4-Isopropylidenimino-3-methyl-1,2,4-triazol-5(4H)-on vom Schmelzpunkt 138 °C.

## Patentansprüche

1. Verfahren zur Herstellung von 4-Amino-1,2,4-triazol-5-onen der Formel (I), 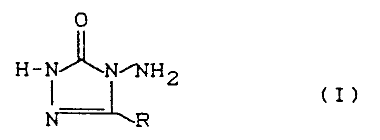 in welcher
R für geradkettiges oder verzweigtes Alkyl mit 1 bis 8 Kohlenstoffatomen steht
dadurch gekennzeichnet, daß man Acylhydrazide der Formel (II), 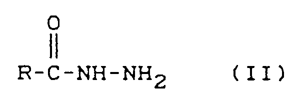 in welcher
R für geradkettiges oder verzweigtes Alkyl mit 1 bis 8 Kohlenstoffatomen steht
mit Phosgen gegebenenfalls in Gegenwart einer Base und gegebenenfalls in Gegenwart eines Verdünnungsmittels umsetzt zu den Oxadiazolinonen der Formel (III), 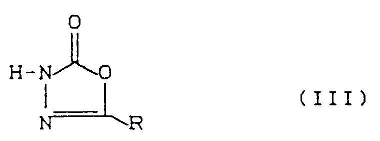 in welcher
R für geradkettiges oder verzweigtes Alkyl mit 1 bis 8 Kohlenstoffatomen steht
und diese Verbindungen der Formel (III) mit Hydrazinhydrat gegebenenfalls in Gegenwart eines Verdünnungsmittels umsetzt.

2. Verfahren gemäß Anspruch 1, dadurch gekennzeichnet, daß man die Umsetzungen in Gegenwart eines Verdünnungsmittels durchführt.

3. Verfahren gemäß Anspruch 2, dadurch gekennzeichnet, daß Benzin, Benzol, Toluol, Xylol, Chlorbenzol, Petrolether, Hexan, Cyclohexan, Dichlormethan, Dichlorethan, Chloroform, Tetrachlorkohlenstoff, Wasser oder Wasser/Toluol oder Wasser/Dichlormethangemisch als Verdünnungsmittel verwendet werden.

4. Verfahren gemäß Anspruch 1, dadurch gekennzeichnet, daß die Umsetzung in Gegenwart von Basen durchgeführt wird.

5. Verfahren gemäß Anspruch 4, dadurch gekennzeichnet, daß Alkalimetallhydroxide, Alkalimetallcarbonate oder -hydrogencarbonate als Basen verwendet werden.

6. Verfahren gemäß Anspruch 1, dadurch gekennzeichnet, daß man die Umsetzung in der ersten Stufe des Verfahrens bei Temperaturen zwischen -20°C und +20°C und in der zweiten Stufe bei Temperaturen zwischen +20°C und +120°C durchführt.

7. Verfahren gemäß Anspruch 1, dadurch gekennzeichnet, daß pro Mol an Acylhydrazid der Formel (II) 1 bis 3 Mol an Phosgen und gegebenenfalls 1 bis 3 Mol an Base sowie 1 bis 10 Mol Hydrazinhydrat eingesetzt werden.

8. Verfahren gemäß Anspruch 1, dadurch gekennzeichnet, daß pro Mol an Acylhydrazid der Formel (II) 1 bis 2 Mol an Phosgen und gegebenenfalls 1 bis 2 Mol an Base sowie 1 bis 5 Mol Hydrazinhydrat eingesetzt werden.

9. Verfahren gemäß Anspruch 1, dadurch gekennzeichnet, daß das Verfahren als Eintopfreaktion durchgeführt wird.

## Claims

1. Process for the preparation of 4-amino-1,2,4-triazol-5-ones of the formula (I) 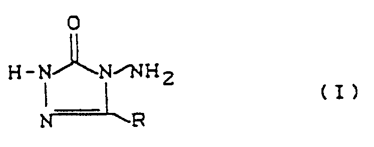 in which
R represents straight-chain or branched alkyl having 1 to 8 carbon atoms,
characterised in that acylhydrazides of the formula (II) 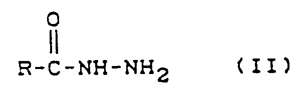 in which
R represents straight-chain or branched alkyl having 1 to 8 carbon atoms,
are reacted with phosgene if appropriate in the presence of a base and if appropriate in the presence of a diluent to give the oxadiazolinones of the formula (III) 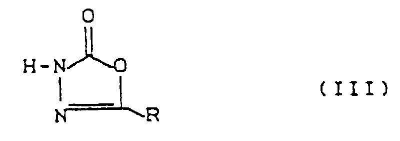 in which
R represents straight-chain or branched alkyl having 1 to 8 carbon atoms,
and these compounds of the formula (III) are reacted with hydrazine hydrate, if appropriate in the presence of a diluent.

2. Process according to Claim 1, characterised in that the reactions are carried out in the presence of a diluent.

3. Process according to Claim 2, characterised in that benzine, benzene, toluene, xylene, chlorobenzene, petroleum ether, hexane, cyclohexane, dichloromethane, dichloroethane, chloroform, carbon tetrachloride, water or water/toluene or water/dichloromethane mixture are used as diluents.

4. Process according to Claim 1, characterised in that the reaction is carried out in the presence of bases.

5. Process according to Claim 4, characterised in that alkali metal hydroxides, alkali metal carbonates or alkali metal hydrogen carbonates are used as bases.

6. Process according to Claim 1, characterised in that the reaction in the first step of the process is carried out at temperatures between -20°C and +20°C and in the second step at temperatures between +20°C and +120°C.

7. Process according to Claim 1, characterised in that 1 to 3 moles of phosgene and if appropriate 1 to 3 moles of base and 1 to 10 moles of hydrazine hydrate are employed per mole of acylhydrazide of the formula (II).

8. Process according to Claim 1, characterised in that 1 to 2 moles of phosgene and if appropriate 1 to 2 moles of base and 1 to 5 moles of hydrazine hydrate are employed per mole of acylhydrazine of the formula (II).

9. Process according to Claim 1, characterised in that the process is carried out in the form of a one-pot reaction.

## Revendications

1. Procédé de production de 4-amino-1,2,4-triazole-5-ones de formule (I), 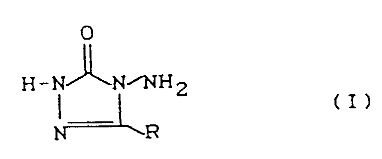 dans laquelle
R est un groupe alkyle à chaîne droite ou ramifiée ayant 1 à 10 atomes de carbone,
caractérisé en ce qu'on fait réagir des acylhydrazides de formule (II), 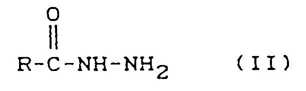 dans laquelle
R est un groupe alkyle à chaîne droite ou ramifiée ayant 1 à 8 atomes de carbone,
avec le phosgène, le cas échéant en présence d'une base et en la présence éventuelle d'un diluant pour former des oxadiazolinones de formule (III), 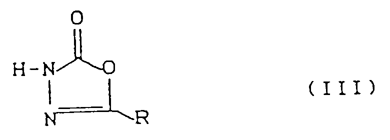 dans laquelle
R est un groupe alkyle à chaîne droite ou ramifiée ayant 1 à 8 atomes de carbone
et on fait réagir ces composés de formule (III) avec l'hydrate d'hydrazine éventuellement en présence d'un diluant.

2. Procédé suivant la revendication 1, caractérisé en ce qu'on conduit les réactions en présence d'un diluant.

3. Procédé suivant la revendication 2, caractérisé en ce qu'on utilise comme diluant l'essence, le benzène, le toluène, le xylène, le chlorobenzène, l'éther de pétrole, l'hexane, le cyclohexane, le dichlorométhane, le dichloréthane, le chloroforme, le tétrachlorure de carbone, l'eau ou un mélange eau/toluène ou eau/dichlorométhane.

4. Procédé suivant la revendication 1, caractérisé en ce qu'on conduit la réaction en présence de bases.

5. Procédé suivant la revendication 4, caractérisé en ce qu'on utilise comme bases des hydroxydes de métaux alcalins, des carbonates et des bicarbonates de métaux alcalins.

6. Procédé suivant la revendication 1, caractérisé en ce qu'on conduit la réaction dans la première étape du procédé à des températures comprises entre -20°C et +20°C et, dans la seconde étape, à des températures comprises entre +20°C et +120°C.

7. Procédé suivant la revendication 1, caractérisé en ce qu'on utilise par mole d'acylhydrazide de formule (II) 1 à 3 moles de phosgène et, le cas échéant, 1 à 3 moles de base ainsi que 1 à 10 moles d'hydrate d'hydrazine.

8. Procédé suivant la revendication 1, caractérisé en ce qu'on utilise par mole d'acylhydrazide de formule (II) 1 à 2 moles de phosgène et, le cas échéant, 1 à 2 moles de base ainsi que 1 à 5 moles d'hydrate d'hydrazine.

9. Procédé suivant la revendication 1, caractérisé en ce que le procédé est mis en oeuvre comme réaction en récipient unique.
